# EUROPEAN PATENT APPLICATION

(11) **EP 3 644 042 A1**
(43) Date of publication of application: **29.04.2020**
(21) Application number: 17914695.6
(22) Date of filing: 23.06.2017
(51) Int. Cl.: G01N 19/02, G01L 5/00

(54) **SLIP TEST DEVICE AND SLIP TEST METHOD**

(71) Applicant: Kaken Test Center, Tokyo 103-0022 (JP)
(72) Inventor: TAKASHIMA Tsuneo, Tokyo 103-0022 (JP); IZUCHI Yukari, Tokyo 103-0022 (JP)
(74) Representative: Rüger Abel Patentanwälte PartGmbB
(86) International application number: PCT/JP2017/023127
(87) International publication number: WO 2018/235249

(57) **Abstract**

A slip test device (1) includes a test member (2), a slip sensor (3), a pressing machine (4), a pressing force meter (5), a holding part (11), and a displacement meter (8). The pressing machine (4) whose movement in a loading direction of the test member is constrained is configured to press a sample (20) against a side of the test member that is an end portion of the test member (2) in a direction perpendicular to the loading direction and with the sample (20) sandwiched between the pressing machine and the side of the test member. The holding part (11) holds the sample (20) between the side of the test member (2) and a pressing part (12). The pressing force meter (5) measures a force with which the sample (20) is pressed against the side of the test member (2). The displacement meter (8) measures the movement of the test member (2) in the loading direction. The slip sensor (3) measures a sliding motion of the sample (20) in the loading direction relative to the pressing part (12) or the test member (2).

## Description

### Technical Field

The present disclosure relates to a slip test device and a slip test method.

### Background Art

Functional materials interposed between two objects and making the action of the boundary surface of each object a desirable property are used in various situations. For example, work gloves are required to have strength, durability, and a fit in accordance with their use and required to generate gripping force or have anti-slip characteristics when an object is gripped. Also, in sports, gloves suitable for gripping and handling a golf club, a racket, a stick, a steering wheel, a handle, a rope, and the like have been developed. In addition, popularly used fabrics include a fabric for adsorbing and removing dirt on a surface of an object such as a lens surface or a glass surface and a fabric for smoothing a surface of an object by applying a coating material such as wax or a lubricant to the surface of the object.

For example, an extent of a slipping motion of the boundary surface is used as an index representing the characteristics of these functional materials. A typical slip characteristic is the coefficient of friction. A method for measuring the coefficient of friction is disclosed in Patent Literature 1, for example. A friction measurement device of Patent Literature 1 includes: pressurizing means for applying a pressing force in a direction perpendicular to a first measurement surface formed by a first measurement object and a second measurement surface formed by a second measurement object in a state where the first and second measurement surfaces are made to come into contact with each other; moving means for relatively moving the first measurement surface and the second measurement surface along these measurement surfaces; and load measuring means for measuring a load at the start of the relative movements of the first and second measurement surfaces and during the relative movements.

Patent Literatures 2 and 3 disclose, as the above-mentioned functional materials, for example, measurement of the friction coefficient of one side of a sample cut out from a glove. Also, Patent Literature 4 discloses measurement of the gripping force of the fabric, because the gripping force is influenced not only by the friction between a target object and a fabric but also by the friction between the fabric and skin.

Regarding characteristics of the boundary surface other than the coefficient of friction, slip detectors are known. Patent Literature 5 discloses an initial slip detecting means having a simple configuration. In a slip detection device of Patent Literature 5, when a contact member comes into contact with a contact receiving member with a pressure-sensitive conductive sheet between the contact member and the contact receiving member, on the basis of a change in a resistance value of the pressure-sensitive conductive sheet that is caused by a detection signal sent from the pressure-sensitive conductive sheet, the slip detection device of Patent Literature 5 is configured to confirm that the initial slip has occurred immediately before occurrence of the slip displacement of the contact member when a high-frequency waveform component occurring immediately before the occurrence of the slip displacement of the contact member exceeds a predetermined threshold.

Patent Literature 6 discloses a slip and slip direction detecting device. In the slip and slip direction detecting device of Patent Literature 6, electrode elements each having two electrodes arranged at a predetermined interval are arranged in a lattice, and a sensor is provided with a pressure-sensitive conductive sheet the resistance value of which changes in accordance with normal force normal to the upper surfaces of the electrode elements. When a high-frequency waveform component occurring due to a sliding motion of a contact member relative to the sensor exceeds a predetermined threshold, an initial slip occurring immediately before the occurrence of the sliding displacement of the contact member relative to the sensor is detected, the center position of a load distribution with respect to the sensor is calculated based on a change in the resistance value of the pressure-sensitive conductive sheet, and the slip direction is simultaneously detected from the direction of the center position of the load distribution when the initial slip is detected.

### Citation List

### Patent Literature

Patent Literature 1: Unexamined Japanese Patent Application Kokai Publication No. 2008-275562
Patent Literature 2: Unexamined Japanese Patent Application Kokai Publication No. 2001-192915
Patent Literature 3: Unexamined Japanese Patent Application Kokai Publication No. 2004-131885
Patent Literature 4: Unexamined Japanese Patent Application Kokai Publication No. 2011-185905
Patent Literature 5: Unexamined Japanese Patent Application Kokai Publication No. 2010-271242
Patent Literature 6: Unexamined Japanese Patent Application Kokai Publication No. 2013-130530

### Summary of Invention

### Technical Problem

In each of the above-mentioned manners of measuring a coefficient of friction, measures the coefficient of friction of one boundary surface is measured. However, for example, evaluation of the feeling of use of a glove cannot be obtained by measuring coefficients of friction of the both sides of the glove in such a manner that measurement of the coefficient of friction of one side of the glove is separated from measurement of the coefficient of friction of the other side of the glove.

In Patent Literature 4, gripping force obtained by combining the friction between the target object and the fabric and the friction between the fabric and the skin is measured. However, in the gripping force measurement of Patent Literature 4, the relative sliding motion between the target object and the fabric and the relative sliding motion between the fabric and the skin cannot be evaluated separately from each other. Even if a result of an evaluation of the gripping force in a case of occurrence of the relative sliding motion between the target object and the fabric is the same as a result of an evaluation of the gripping force in a case of occurrence of the relative sliding motion between the fabric and the skin in the device of Patent Literature 4, the actual feeling of use of the fabric in the case in which the relative sliding motion occurs mainly between the target object and the fabric is expected to differ from the actual feeling of use of the fabric in the case in which the relative sliding motion occurs mainly between the fabric and the skin.

In consideration of the aforementioned circumstances, an objective of the present disclosure is to simultaneously measure sliding motions of both sides of a sample.

### Solution to Problem

A slip test device according to a first aspect of the present disclosure includes: a test member; a pressing mechanism whose movement in a loading direction of the test member is constrained, the pressing mechanism being configured to press a sample against an end surface of the test member that is an end portion of the test member in a direction perpendicular to the loading direction with the sample sandwiched between the end surface of the test member and the pressing mechanism; a holding part to hold the sample between the end surface of the test member and the pressing mechanism; a pressing force meter to measure a pressing force with which the pressing mechanism presses the sample against the end surface of the test member; a displacement meter to measure a movement of the test member in the loading direction; and a slip measurer to measure a sliding motion of the sample in the loading direction relative to the pressing mechanism or the test member.

A slip test method according to a second aspect of the present disclosure includes: pressing a sample against an end surface of a test member in a direction perpendicular to a loading direction of the test member using a pressing mechanism whose movement in the loading direction of the test member is constrained, the end surface being an end portion of the test member in the direction perpendicular to the loading direction; measuring, using a pressing force meter, a force with which the pressing mechanism presses the sample against the end surface of the test member; and while decreasing a pressing force having such magnitude as does not cause the test member to move in the loading direction after the pressing force is applied to a boundary portion between the end surface and the sample by the pressing mechanism, measuring, using a displacement meter, the movement of the test member in the loading direction and measuring, using a slip measurer, a sliding motion of the sample in the loading direction relative to the pressing mechanism or the test member.

### Advantageous Effects of Invention

According to the present disclosure, sliding motions of both sides of the sample can be simultaneously measured.

### Brief Description of Drawings

FIG. 1 is a view illustrating a configuration of a slip test device according to Embodiment 1 of the present disclosure;
FIG. 2 is a block diagram illustrating the slip test device according to Embodiment 1;
FIG. 3 is a conceptual diagram illustrating results of a slip test in Embodiment 1;
FIG. 4 is a view illustrating a configuration of a slip test device according to Embodiment 2 of the present disclosure;
FIG. 5 is a view illustrating a configuration of a slip test device according to Embodiment 3 of the present disclosure;
FIG. 6 is a block diagram illustrating the slip test device according to Embodiment 3;
FIG. 7 is a flowchart illustrating an example of a process of a slip test in Embodiment 3;
FIG. 8 is a flowchart illustrating an example of a process of a slip test in Embodiment 3 including repeated steps;
FIG. 9 is a view illustrating a configuration of a slip test device according to Embodiment 4 of the present disclosure;
FIG. 10 is a block diagram illustrating the slip test device according to Embodiment 4;
FIG. 11 is a flowchart illustrating an example of a process of a slip test in Embodiment 4;
FIG. 12 is a view illustrating a configuration of a slip test device according to Embodiment 5 of the present disclosure;
FIG. 13 is a cross-sectional view illustrating a configuration of the slip test device according to Embodiment 5; and
FIG. 14 is a view illustrating a configuration of a slip test device according to Embodiment 6 of the present disclosure.

### Description of Embodiments

Embodiments of the present disclosure are described below in detail with reference to the drawings. In the drawings, components that are the same or equivalent are assigned the same reference signs.

### Embodiment 1

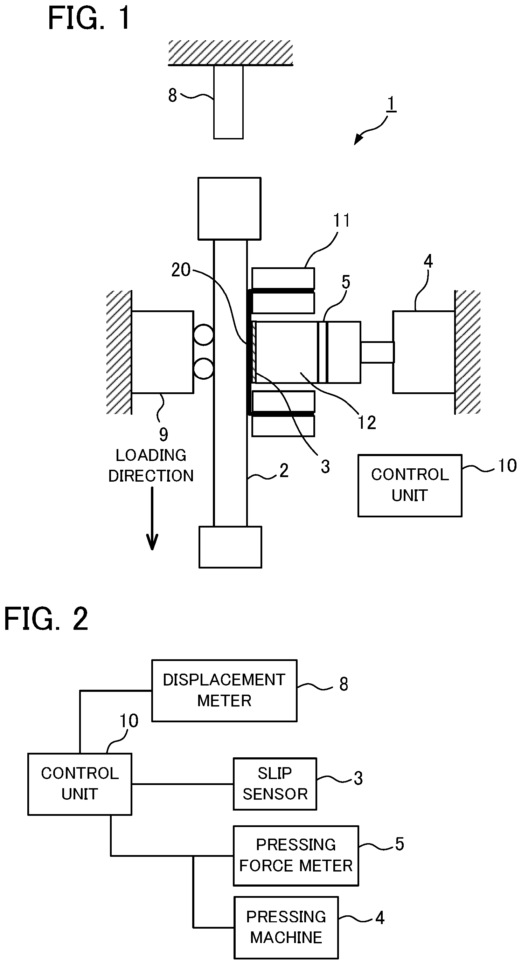

FIG. 1 is a view illustrating a configuration of a slip test device according to Embodiment 1 of the present disclosure. In FIG. 1, the downward direction is the direction of gravity. A slip test device 1 includes a test member 2, a holding part 11, a slip sensor 3, a pressing part 12, a pressing force meter 5, a pressing machine 4, an opposite unit 9, and a displacement meter 8. The pressing part 12, the pressing machine 4, and the opposite unit 9 constitute a pressing mechanism. The pressing mechanism is supported by a housing not illustrated in the drawings, and the pressing mechanism is constrained at least in the vertical direction and thus does not move in the vertical direction. For example, the holding part 11 is supported in balance with a balancer not illustrated in the drawings and can be moved freely in the vertical direction. However, the holding part 11 does not move under its own weight.

The sample 20 is typically a sheet, and the holding part 11 holds a sample 20, for example, by sandwiching two opposite edges of the sample 20 by the holding part. For example, the slip test device of Embodiment 1 may have a structure for adjusting a distance between frames between which the two edges of the sample are sandwiched so that tension can be applied to the sample 20. Alternatively, the holding part may be configured to sandwich the outer periphery of the sample 20 between two rings in a manner similar to a manner of stretching a drumhead upon a drum. In that case, tension may be applied to the sample 20 by adjusting amounts by which the two rings are pushed.

The sample 20 that is an object to be subjected to the slip test is, for example, (i) a sheet cut out from a glove or a sock, (ii) a cloth for removing dirt, or (iii) a polishing cloth. The sample 20 is held by the holding part 11 and is pressed against the test member 2 by a pressing mechanism with the slip sensor 3 interposed between the sample 20 and the pressing part. For example, the holding part 11 holds the sample 20 in such a manner that the sample 20 can move in the loading direction relative to the test member 2 and the pressing mechanism. In Embodiment 1, the test member 2 is held by the frictional force with the sample 20 caused by pressing force, and the sample 20 slides downward due to gravity when the frictional force becomes less than a weight of the sample. Gravity acts on the test member 2, and the direction of gravity is the loading direction. The test member 2 is a column whose cross section is shaped like, for example, a polygon, a circle or an ellipse, or an arbitrary closed curve. The loading direction is the direction of the columnar central shaft. The opposite unit 9 is in contact with the test member 2 via a roller that is rotatably supported, and faces the sample 20 with the test member 2 between the opposite unit 9 and the sample 20. The opposite unit 9 allows the test member 2 to move in the direction of gravity and maintains the position of the test member 2 in a direction perpendicular to the direction of gravity against the force pressing the sample 20 and the slip sensor 3.

The pressing machine 4 may be composed of, for example, a motor and a ball screw. In that case, the pressing machine 4 and the opposite unit 9 are fixed to the housing, and the pressing machine 4 presses the test member 2 against the opposite unit 9. Although FIG. 1 illustrates a structure in which the pressing machine 4 and the opposite unit 9 are fixed, the present disclosure is not limited to such a structure. For example, the present disclosure may use two parallel and opposite racks and a single pinion that are arranged in such a manner that the two racks mesh with the pinion and move in directions opposite to each other. The pressing part 12 and the opposite unit 9 may be fixed to racks that move in directions opposite to each other, and the racks may be moved by rotation of a pinion so that the test member 2 is sandwiched between the pressing part 12 and the opposite unit 9. In that case, a motor that rotates the pinion, the racks, and the pinion constitute the pressing machine 4.

The pressing force meter 5 is interposed between the pressing machine 12 and the pressing machine 4 and measures a pressing a force with which the pressing mechanism presses the sample 20 against the test member 2. The value of the pressing force measured by the pressing force meter 5 is fed back to the pressing machine 4, and the pressing machine 4 is controlled to press the sample with a commanded pressing force.

The slip sensor 3 serving as a slip measurer is pressed against the test member 2 together with the sample 20 by the pressing mechanism. In the configuration illustrated in FIG. 1, the slip sensor 3 is fixed to the pressing part 12 and detects a sliding motion of the sample 20 relative to the slip sensor. The order of the arrangement of the slip sensor 3 and the sample 20 may be reversed. That is, the present disclosure may use a structure in which the slip sensor 3 is fixed to the test member 2, the pressing part 12 comes into contact with the sample 20, and the sample 20 and the slip sensor 3 are pressed against the test member 2. Even in that case, the slip sensor 3 detects the sliding motion of the sample 20 relative to the slip sensor 3.

For example, the slip detection member composed of the pressure-sensitive conductive sheet and the electrode sheet described in Patent Literature 5 may be used as the slip sensor 3. Alternatively, the sensor part composed of the pressure-sensitive conductive sheet and electrode part described in Patent Literature 6 may be used as the slip sensor 3. In a case in which the slip sensor 3 is fixed to the pressing part 12, the slip sensor 3 measures a sliding motion of the sample 20 in the loading direction relative to the pressing part 12 that is a component of the pressing mechanism. In a case in which the slip sensor 3 is fixed to the test member 2, the slip sensor 3 detects a sliding motion of the sample 20 in the loading direction relative to the test member 2.

The displacement meter 8 measures a movement of the test member 2 in the vertical direction, that is, in the loading direction. The displacement meter 8 is, for example, a non-contact laser displacement meter. The movement of the test member 2 in the loading direction measured by the displacement meter 8, that is, the displacement of the test member 2, is the sum of (i) a slip amount by which the sliding motion of the sample 20 slides relative to the test member 2 and (ii) a slip amount by which the sample 20 slides relative to the pressing mechanism. Therefore, the difference between the displacement measured by the displacement meter 8 and the slip amount of measured by the slip sensor 3 is the slip amount of the sample 20 relative to the test member 2.

In a case in which the order of the arrangement of the sample 20 and the slip sensor 3 is reversed to be opposite to the order of the arrangement illustrated in FIG. 1 and thus the slip sensor 3 is fixed to the test member 2, the displacement measured by the displacement meter 8 is the sum of (i) a slip amount by which the sample 20 slides relative to the slip sensor 3 and (ii) a slip amount by which the sample 20 slides relative to the pressing part 12. In that case, the slip amount of the sample 20 relative to the pressing part 12 is a difference between the displacement measured by the displacement meter 8 and the slip amount of the sample measured by the slip sensor 3.

In order to perform the test, the test member 2 is first held in the position illustrated in FIG. 1 by some means, the pressing part 12 is moved backward toward the pressing machine 4, the holding part 11 is removed, and the sample 20 is attached to the holding part 11. The sample 20 is disposed so as to come into contact with the end surface (side surface) of the test member 2 in the direction perpendicular to the loading direction, and the pressing machine 12 is pressed against the sample 20 by the pressing machine 4. After confirming that the test member 2 does not move even if the act of holding the test member 2 in some means is stopped, the pressing force pressed by the pressing machine 4 is changed, and the displacement and the slip amounts are measured.

In order to hold the test member 2 during attachment of the sample 20, for example, the test member 2 may be suspended by using a wire and a pulley to hang the test member 2 and braking the pulley over which the wire runs. After setting the sample 20, the brake on the pulley is released so that the test member 2 can fall freely.

FIG. 2 is a block diagram illustrating the slip test device according to Embodiment 1. The slip sensor 3, the pressing force meter 5, the pressing machine 4, and the displacement meter 8 are connected to the control unit 10, operate in response to commands from the control unit 10, and send measurement values to the control unit 10. The control unit 10 is constituted by a computer, for example. The control unit 10 controls the pressing machine 4 so that the pressing force becomes a commanded value, measures the movement of the test member 2 in the loading direction using the displacement meter 8, and measures the slip amount of the sample 20 relative to the slip sensor 3. The control unit 10 may merely output, to the display unit or the outside, the commanded pressing force, the displacement, and the slip amount or may store them in a storage unit.

Additionally, the control unit 10 may change the pressing force produced by the pressing machine 4 on the basis of some rules, thereby determining what pressing force causes the movement of the test member 2 in the loading direction and the sliding motion of the sample. For example, pressing force causing the movement of the test member 2 in the loading direction and the sliding motion of the sample can be determined by first applying a pressing force that does not cause a movement of the test member 2 and reducing the pressing force. Since the difference between the displacement of the test sample and the slip amount relative to the pressing mechanism is the slip amount of the sample 20 relative to the test member 2, the sliding motions of both surfaces of the sample 20 can be measured simultaneously. By comparing the sensory test of the use of the sample 20 with the measurement results from the slip test device 1, the relationship between the sensory evaluation of the sample 20 and the slip characteristics on both surfaces of the sample can be examined.

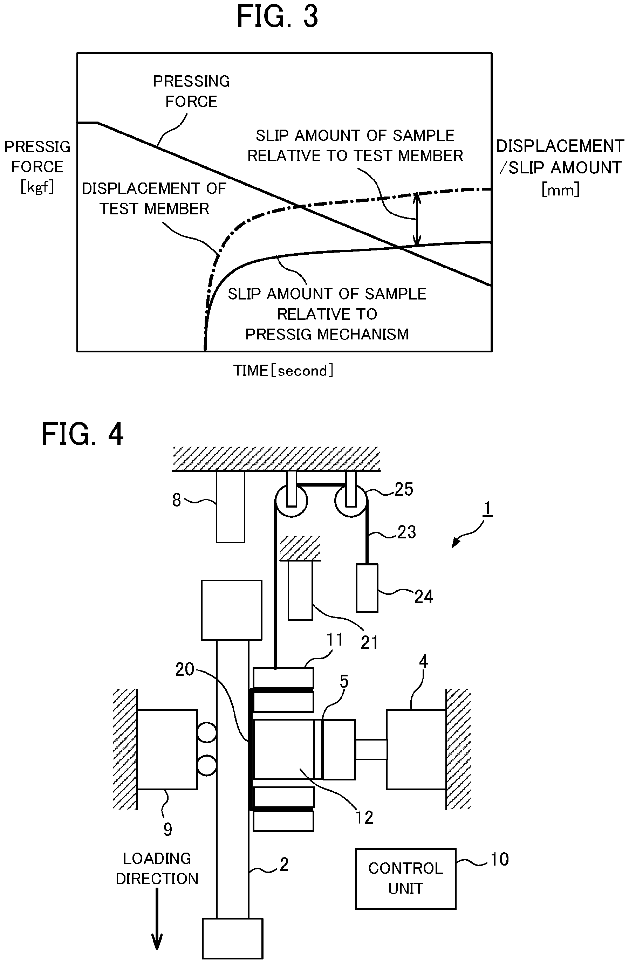

FIG. 3 is a conceptual diagram illustrating results of the slip test in Embodiment 1. FIG. 3 illustrates an example in which the slip sensor 3 is fixed to the pressing part 12. The pressing force that does not cause the movement of the test member 2 is first applied, and the pressing force is reduced from that state. Then, the displacement of the test member 2 starts to occur at some point in time, and the sample 20 slides relative to the slip sensor 3 fixed to the pressing mechanism simultaneously with or after the occurrence of the displacement of the test member 2. A difference obtained by subtracting the slip amount of the sample 20 relative to the pressing mechanism from the displacement of the test member 2 is the slip amount of the sample 20 relative to the test member 2. The displacement of the test member 2 does not always starts to occur simultaneously with the occurrence of the sliding motion of the sample 20 relative to the pressing mechanism.

Although the test member 2 has a columnar shape in Embodiment 1, the shape of the test member 2 is not limited to the columnar shape. For example, a grip to which a tape is helically attached, a braided rope or a twisted rope, a sphere similar to a ball, or a spheroid similar to an egg may be used in accordance with the actual use of the sample 20. In that case, a surface of the test member that comes into contact with the opposite unit 9 is preferably formed to be a column-shaped lateral surface so that the test member moves linearly relative to the opposite unit 9. The column-shaped lateral surface is a surface formed by moving, along the cross-sectional shape, a straight line parallel to the loading direction. Further, the pressing part 12 may be an elastic body so that the sample 20 and the slip sensor 3 fit the surface of the test member 2.

### Embodiment 2

FIG. 4 is a view illustrating a configuration of a slip test device according to Embodiment 2 of the present disclosure. In Embodiment 2, a sample displacement meter 21 is used instead of the slip sensor 3. In Embodiment 2, an end portion of the holding part 11 is fixed to one end of the wire 23 and is hung from the wire 23. A weight 24 having the same mass as the holding part 11 and the sample 20 is fixed to the other end of the wire 23. The wire 23 runs over the pulley 25, and the holding part 11 can be freely moved in the vertical direction. Since the holding part 11 and the sample 20 are balanced with the weight 24, the holding part 11 does not move under its own weight. The holding part 11 may be configured to apply tension to the sample 20.

The sample displacement meter 21 measures a movement of the holding part 11 holding the sample 20 in the vertical direction, that is, in the loading direction. The sample displacement meter 21 is, for example, a non-contact laser displacement meter. The movement of the holding part 11 in the loading direction measured by the sample displacement meter 21 is the sliding motion of the sample 20 relative to the pressing machine 12 that is a component of the pressing mechanism. The sample displacement meter 21 is a slip measurer.

In Embodiment 2, a sample displacement meter 21 is connected to the control unit 10 instead of the slip sensor 3. The sample displacement meter 21 measures the sliding motion of the sample 20 relative to the pressing mechanism and sends a measured value to the control unit 10. The control unit 10 calculates a slip amount of the sample 20 relative to the test member 2 by subtracting a slip amount measured by the sample displacement meter 21 from a displacement of the test member 2 measured by the displacement meter 8. Since the difference between the displacement of the test member 2 and the slip amount measured by the sample displacement meter 21 is the slip amount of the sample 20 relative to the test member 2, the sliding motions of the both surfaces of the sample 20 can be measured simultaneously.

In Embodiment 2, both the sample displacement meter 21 and the slip sensor 3 may be used. Alternatively, the slip sensor 3 may be fixed to the test member 2 and the sliding motion of the sample 20 relative to the test member 2 may be measured by the slip sensor 3. In that case, since the sample displacement meter 21 can measure the sliding motion of the sample 20 relative to the pressing mechanism, the sliding motions of the both surfaces of the sample 20 can be simultaneously measured without the displacement meter 8. In this case, the slip sensor 3 fixed to the test member 2 can be regarded as a displacement meter that measures the movement of the test member 2 in the loading direction. The sample displacement meter 21 is a slip measurer that measures the sliding motion of the sample 20 relative to the pressing mechanism.

### Embodiment 3

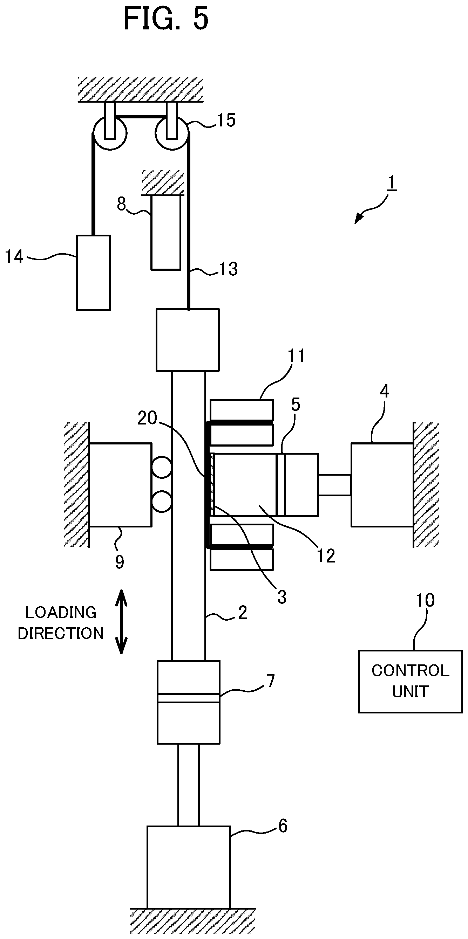

FIG. 5 is a view illustrating a configuration of a slip test device according to Embodiment 3 of the present disclosure. In Embodiment 3, the test member 2 is suspended and supported, and the slip test device of Embodiment 3 includes a loading machine 6 applying a force in the loading direction. The other configurations of Embodiment 3 are the same as those of Embodiment 1. Also in Embodiment 3, the holding part 11 may be configured to apply tension to the sample 20.

The test member 2 according to Embodiment 3 has an end fixed to one end of the wire 13 and is hung from the wire 13. A weight 14 having the same mass as the test member 2 is fixed to the other end of the wire 13. The wire 13 runs over the pulley 15, and the test member 2 can be freely moved in the vertical direction. Since the test member 2 are balanced with the weight 14, the test member 2 does not move under its own weight.

The loading machine 6 is connected to the end of the test member 2 opposite to the end fixed to the wire 13 via a load meter 7. The loading machine 6 can apply a vertically downward force or a vertically upward force to the test member 2. The loading direction is a vertically downward direction or a vertically upward direction. The loading machine 6 may be composed of, for example, a motor and a ball screw. The load meter 7 measures a force that is applied to the test member 2 by the loading machine 6.

Since the test member 2 is suspended and supported and the position of the test member 2 in the horizontal plane is fixed to a certain position in Embodiment 3, the slip test device of Embodiment 3 is desirably configured to have a structure enabling adjustment of the position of the opposite unit 9 in accordance with the test member 2. In a case in which the pressing mechanism includes two opposite and parallel racks and a single pinion and the two racks mesh with the pinion and move in directions opposite to each other, the pressing mechanism may be configured to have a structure in which the whole of the pressing mechanism including the motor, the racks and the pinion can freely move in the direction of the pressing force.

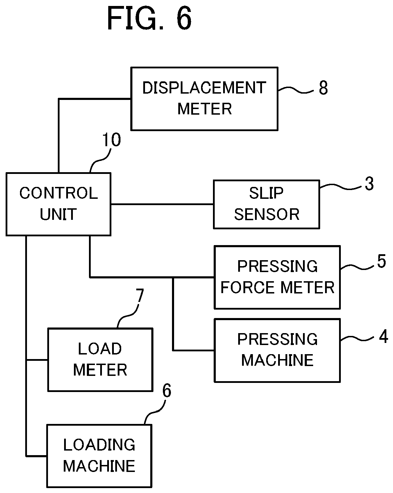

FIG. 6 is a block diagram illustrating the slip test device according to Embodiment 3. In addition to the configurations of Embodiment 1, the load meter 7 and the loading machine 6 are connected to the control unit 10. The load value measured by the load meter 7 is fed back to the loading machine 6, and the loading machine 6 is controlled to apply a commanded force to the test member 2.

In Embodiment 3, since the test member 2 is suspended and balanced with the weight 14, the test member 2 does not move unless force is applied by the loading machine 6. In a state where no force is applied by the loading machine 6, the sample 20 can be attached and a pressing force can be applied by the pressing machine 4. The movement in the loading direction and the slip amount can be measured while reducing the pressing force after applying the force in the loading direction by the loading machine 6 with the pressing force applied.

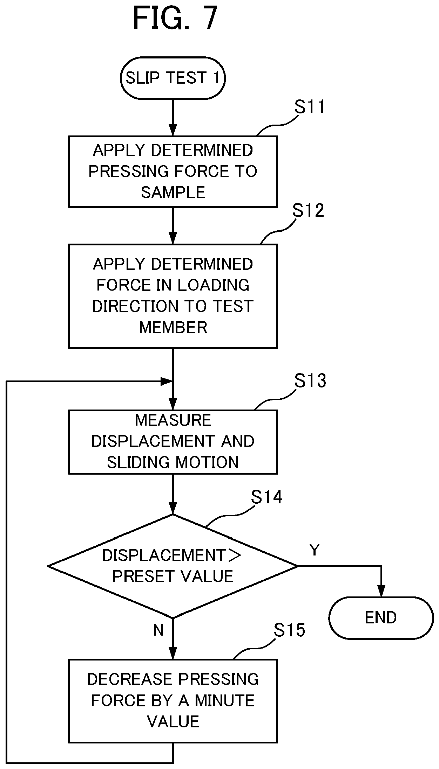

FIG. 7 is a flowchart illustrating an example of a process of a slip test in Embodiment 3. The sample 20 is attached to the slip test device 1 and the slip test is started. The controller 10 applies a commanded and determined pressing force to the sample 20 (Step S11). A determined force in the loading direction is applied by the loading machine 6 with the determined pressing force applied (Step S12). For example, a constant force that does not cause a movement of the test member 2 is applied as the commanded pressing force. In this state, the displacement and the sliding motion are measured (Step S13).

If the displacement is equal to or less than the predetermined value (No in Step S14), the pressing force is decreased by a minute value (Step S15), and the displacement and the sliding motion are measured (Step S13). The step of measuring the displacement and the sliding motion after reducing the pressing force by the minute value is repeated, and if the displacement becomes greater than the predetermined value (Yes in Step S14), the test is terminated. The condition for determining whether the test is terminated may be (i) the number of times that the step of decreasing the pressing force by the minute value is performed, (ii) time taken to perform the step of decreasing the pressing force by the minute value, or (iii) a condition that the slip amount measured by the slip sensor 3 exceeds a predetermined value.

Since the force in the loading direction can be freely set independently of the mass of the test member 2 in Embodiment 3, a testable range in Embodiment 3 is wider than that in Embodiment 1. Also, the force in the loading direction can be changed during the test. For example, the occurrence of the sliding motion can be tested by increasing the force in the loading direction applied by the loading machine 6 while the pressing force applied by the pressing machine 4 is being kept constant. Furthermore, since the loading direction can be reversed, an operation in which the direction of the applied force is reversed can be simulated.

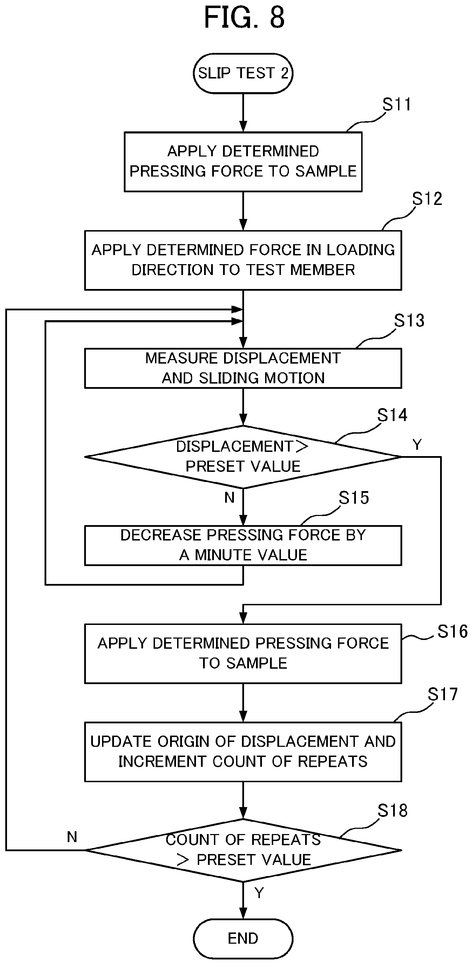

FIG. 8 is a flowchart illustrating an example of a process of a slip test in Embodiment 3 including repeated steps. In the slip test illustrated in FIG. 8, the steps of measuring the displacement and the sliding motion are repeated while the pressing force is being decreased. The operations from Step S11 to Step S15 in FIG. 8 are the same as those in the slip test in FIG 7. When the displacement exceeds a predetermined value (Yes in Step S14), a determined pressing force that is commanded anew is applied to the sample 20 (Step S16). The pressing force determined in this case may be changed from the pressing force first applied to the sample. Then, the position of the test member 2 at that time is set as the origin of the displacement, and the count of repeats of the steps is incremented (Step S17).

If the count of repeats of the steps is equal to or less than a predetermined value (No in Step S18), the process returns to Step S13 to measure the displacement and the sliding motion (Step S13). When the count of repeats of the steps exceeds the predetermined value (Yes in Step S18), the test is terminated.

In the slip test illustrated in FIG. 8, a re-gripping operation performed when the sliding motion is detected can be simulated. Also, for example, in a case in which the sample 20 is a low resilient material, the behavior of the sample can be examined when the step of pressing the sample is repeated.

In the slip test illustrated in FIG. 8, the condition for determining whether the anew determined pressing force is applied to the sample is not limited to the condition that the displacement exceeds a predetermined value, and may be (i) the number of times that the step of decreasing the pressing force by the minute value is performed, (ii) time taken to perform the step of decreasing the pressing force by the minute value, or (iii) a condition that the amount of the slip amount measured by the slip sensor 3 exceeds a predetermined value. Alternatively, the determined pressing force may be changed as often as the steps are repeated. For example, the pressing force may be changed so as to be gradually increased or gradually decreased or may be changed randomly within a certain range.

In Embodiment 3, the loading machine 6 may apply, to the sample 2, not only the force in the vertical direction but also a torque for rotating the test member 2 around the axis in the vertical direction. In that case, the test member 2 is desirably a cylinder. A portion of the test member 2 that comes into contact with the opposite unit 9 is at least formed to be a cylindrical surface. The opposite unit 9 comes into contact with the test member 2 via a ball that can rotate in all directions instead of the roller, and the opposite unit 9 maintains a position of the test member 2 in a direction perpendicular to the loading direction. The opposite unit 9 includes at least three balls arranged at the vertices of the triangle. The torque for rotating the test member 2 is applied to the test member, thereby simulating an operation of pulling or pushing the sample while twisting the sample.

Also in Embodiment 3, the sample displacement meter 21 may be used instead of the slip sensor 3 as in Embodiment 2. Additionally, the slip sensor 3 and the sample displacement meter 21 may be used in combination. Alternatively, the sliding motion of the sample 20 relative to the pressing mechanism may be measured by the sample displacement meter 2, and the sliding motion of the sample 20 relative to the test member 2 may be measured by the slip sensor 3 with the slip sensor 3 fixed to the test member 2. In this case, the slip sensor 3 fixed to the test member 2 can be regarded as a displacement meter that measures the movement of the test member 2 in the loading direction. The sample displacement meter 21 is a slip measurer that measures the sliding motion of the sample 20 relative to the pressing mechanism.

### Embodiment 4

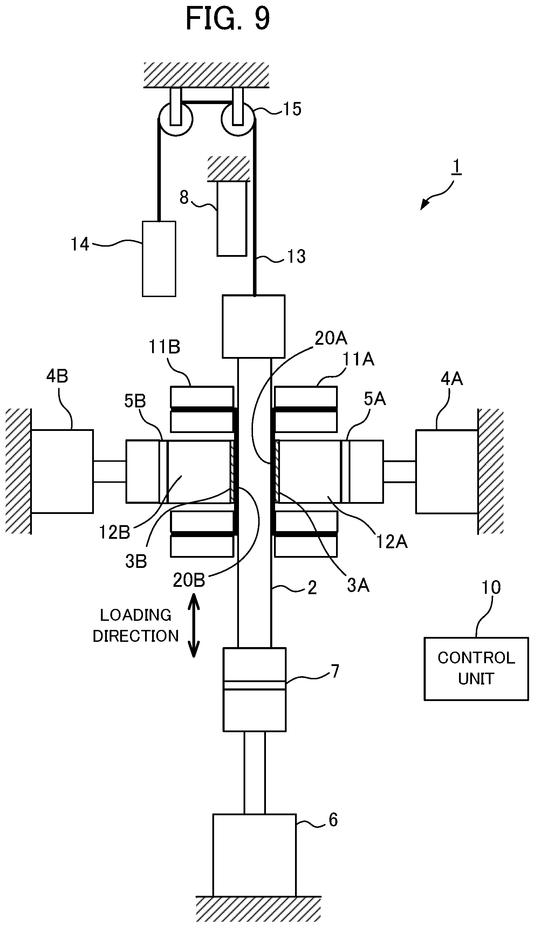

FIG. 9 is a view illustrating a configuration of a slip test device according to Embodiment 4 of the present disclosure. The slip test device 1 according to Embodiment 4 includes two slip sensors 3A and 3B that face each other with the test member 2 interposed between the slip sensors 3A and 3B. The slip test device 1 includes two holding parts 11A and 11B that respectively correspond to the slip sensors 3A and 3B and that respectively hold the samples 20A and 20B. In the configuration illustrated in FIG. 9, the pressing mechanism includes two pressing machines 4A and 4B, two pressing force meters 5A and 5B, and two pressing parts 12A and 12B. The sample 20A and the slip sensor 3A are pressed against one end surface of the test member 2 in the direction perpendicular to the loading direction, and the sample 20B and the slip sensor 3B are pressed against the other end surface of the test member 2 in the direction perpendicular to the loading direction.

The slip test device 1 according to Embodiment 4 has a configuration in which the holding part 11B, the slip sensor 3B, the pressing part 12B, the pressing force meter 5B, and the pressing machine 4B are used instead of the opposite unit 9 of Embodiment 3. The test member 2 and a suspension support mechanism for supporting the test member 2, the displacement meter 8, the load meter 7, and the loading machine 6 are the same as those of Embodiment 3. Also in Embodiment 4, the holding parts 11A and 11B may be configured to respectively apply tension to the samples 20A and 20B.

In the slip test device 1 illustrated in FIG. 9, the operations of the two pressing machines 4A and 4B are desirably controlled to be adjusted so that the position of the test member 2 in the pressing direction perpendicular to the loading direction does not deviate from a reference position.

Although the pressing mechanism illustrated in FIG. 9 includes two pressing machines 4A and 4B that operate independently of each other, the pressing mechanism may be formed as a single pressing mechanism constituted by (i) two racks that are parallel and opposite to each other and (ii) a single pinion with which the two racks mesh to move in directions opposite to each other. The slip test device 1 of Embodiment 4 may have a configuration in which the pressing part 12A and the pressing part 12B are fixed to the racks that move in directions opposite to each other and the racks are moved by the pinion, thereby sandwiching the test member 2 between the pressing parts 12A and 12B. In a case in which the pressing mechanism includes the racks and the pinion, the pressing mechanism may be configured in such a manner that the whole of the pressing mechanism including the motor, the racks and the pinion can freely move in the direction of the pressing force.

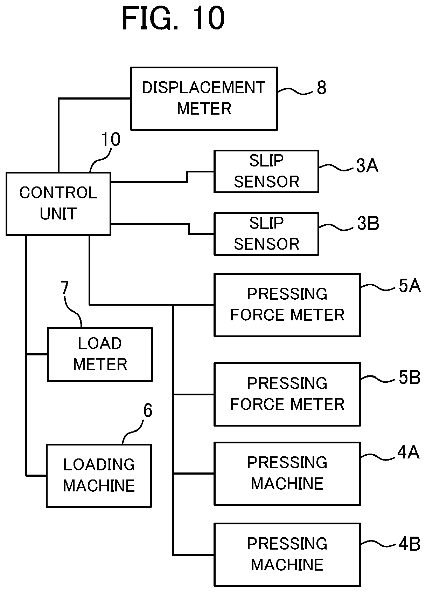

FIG. 10 is a block diagram illustrating the slip test device according to Embodiment 4. Since the two slip sensors 3A and 3B, the two pressing force meters 5A and 5B, and the two pressing machines 4A and 4B are included in the slip test device according to Embodiment 4, the slip sensors 3A and 3B, the pressing force meters 5A and 5B, and the pressing machines 4A and 4B are connected to the control unit 10, operate in response to commands from the control unit 10, and send measurement values to the control unit 10.

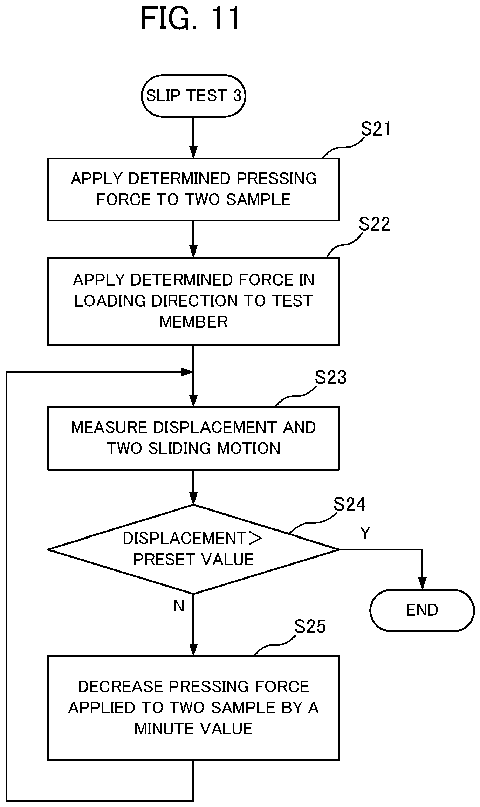

FIG. 11 is a flowchart illustrating an example of a process of a slip test in Embodiment 4. The process of the slip test illustrated in FIG. 11 is a process to which the process illustrated in FIG. 7 is expanded so as to be applicable to a case in which the two slip sensors 3A and 3B are included in the slip test device. Since the slip sensors 3A and 3B are pressed against the test member 2 with the same pressing force, there is no difference from the process of Embodiment 3 except that sliding motions of the samples are measured by the two slip sensors 3A and 3B.

The samples 20A and 20B are attached to the slip test device 1, and the slip test is started. The controller 10 applies commanded predetermined pressing forces to the samples 20A and 20B (Step S21). In a state in which the determined pressing forces are applied to the samples, a determined force in the loading direction is applied to the test member by the loading machine 6 (Step S22). For example, a constant force that does not cause movement of the test member 2 is applied to the test member 2 with the commanded pressing force. In this state, the displacement and the sliding motions are measured (Step S23).

If the displacement is equal to or less than a predetermined value (No in Step S24), the pressing forces applied to the two samples 20A and 20B are decreased by a minute value (Step S25), and the displacement and the sliding motions are measured (Step S23). The step of measuring the displacement and the sliding motions by decreasing the pressing force by the minute value is repeated, and when the displacement becomes greater than the predetermined value (Yes in Step S24), the test is terminated. The condition for determining whether the test is terminated may be (i) the number of times that the step of decreasing the pressing force by the minute value is performed, (ii) time taken to perform the step of decreasing the pressing force by the minute value, or (iii) a condition that the slip amount measured by the slip sensor 3A or 3B exceeds a predetermined value.

Also in the configuration illustrated in Embodiment 4, as in the step of Embodiment 3 illustrated in FIG. 8, the step of measuring the displacement and the sliding motions can be repeatedly perform while reducing the pressing forces.

Also in Embodiment 4, the loading machine 6 may apply, to the test member 2, not only the force in the vertical direction but also a torque for rotating the test member 2 around the axis in the vertical direction. In Embodiment 4, the test member 2 is not limited to a cylinder. For example, a grip to which a tape is helically attached or a braided rope or a twisted rope may be used. In Embodiment 4, a joint portion between the wire 13 hanging the test member 2 and the test member 2 can be desirably rotated around a vertical axis. The torque for rotating the test member 2 is applied to the test member 2, thereby simulating an operation of pulling or pushing the sample while twisting the sample.

Also in Embodiment 4, the sample displacement meter 21 may be used in place of at least one of the slip sensors 3A and 3B as in Embodiment 2. Alternatively, the slip sensors 3A and 3B and the sample displacement meter 21 may be used in combination with one another. Alternatively, the sliding motion of the sample 20A or 20B relative to the pressing mechanism may be measured by the sample displacement meter 21, and the sliding motion of the sample 20A or 20B relative to the test member 2 may be measured by the slip sensor 3A or 3B with the slip sensor 3A or 3B fixed to the test member 2. In this case, the slip sensor 3A or 3B fixed to the test member 2 can be regarded as a displacement meter that measures the movement of the test member 2 in the loading direction. The sample displacement meter 21 is a slip measurer that measures the sliding motion of the sample 20A or 20B relative to the pressing mechanism.

### Embodiment 5

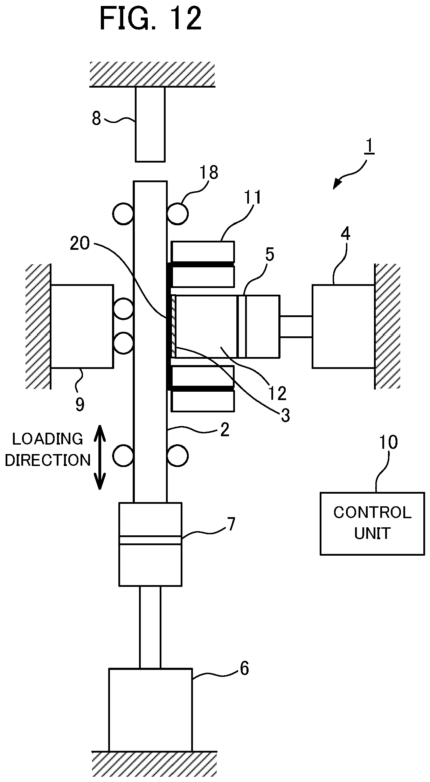

FIG. 12 is a view illustrating a configuration of a slip test device according to Embodiment 5 of the present disclosure. FIG. 12 is a plan view of the slip test device 1 as viewed from above. In Embodiment 5, the test member 2 is supported in such a manner that the test member 2 is movable in the horizontal direction. The loading direction of the test member 2 is the horizontal direction. Except that the test member 2 is supported in such a manner that the test member 2 is movable in the horizontal direction, the slip test device 1 of Embodiment 5 has the same configuration as in Embodiment 3. The pressing direction of the pressing machine 4 and the loading direction of the loading machine 6 are both horizontal and are perpendicular to each other. The test member 2 is guided by a roller 18 and can freely move in the loading direction of the loading machine 6.

In the slip test device 1 according to Embodiment 5, although the holding part 11 can be freely moved in the horizontal direction, the holding part 11 is desirably supported so as not to fall under its own weight. Also in Embodiment 5, the holding part 11 may be configured to apply tension to the sample 20.

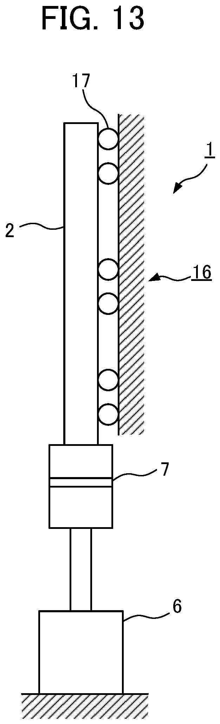

FIG. 13 is a cross-sectional view illustrating a configuration of the slip test device according to Embodiment 5. FIG. 13 is a cross-sectional view of the slip test device 1 as viewed in the pressing direction of the pressing machine 4. The test member 2 is supported by a support portion 16 in such a manner that the test member 2 is movable in the horizontal direction via a roller 17. At least a surface of the test member coming into contact with the roller 17 is desirably a columnar side surface so that the test member 2 can move linearly in the loading direction.

Since the test member 2 does not move unless a load is applied to the test member, a process of the slip test in Embodiment 5 is the same as that in Embodiment 3. Also in the slip test device 1 according to Embodiment 5, as in the process according to Embodiment 3 illustrated in FIG. 8, the step of measuring the displacement and the sliding motion can be repeatedly performed while the pressing force is being decreased.

In the slip test device 1 according to Embodiment 5, as in Embodiment 3, the loading machine 6 may apply, to the test member, not only the force in the loading direction but also a torque for rotating the test member 2 around the axis in the loading direction. In that case, the test member 2 is desirably a cylinder. Portions of the test member 2 that come into contact with the support portion 16 and the opposite unit 9 are at least formed to be a cylindrical surface. Additionally, instead of the roller, the support portion 16 and the opposite unit 9 come into contact with the test member 2 via the ball that can rotate in all directions and maintain the position of the test member 2 in the direction perpendicular to the loading direction.

Also in Embodiment 5, the sample displacement meter 21 may be used in place of the slip sensor 3 as in Embodiment 2. In that case, the sample displacement meter 21 is arranged in the horizontal direction and measures a sliding motion of the holding part 11 relative to the pressing mechanism in the horizontal direction that is the loading direction. Alternatively, the slip sensor 3 and the sample displacement meter 21 may be used in combination. Alternatively, the sliding motion of the sample 20 relative to the pressing mechanism may be measured by the sample displacement meter 21, and the sliding motion of the sample 20 relative to the test member 2 may be measured by the slip sensor 3 with the slip sensor 3 fixed to the test member 2. In this case, the slip sensor 3 fixed to the test member 2 can be regarded as a displacement meter that measures the movement of the test member 2 in the loading direction. The sample displacement meter 21 is a slip measurer that measures the sliding motion of the sample 20 relative to the pressing mechanism.

### Embodiment 6

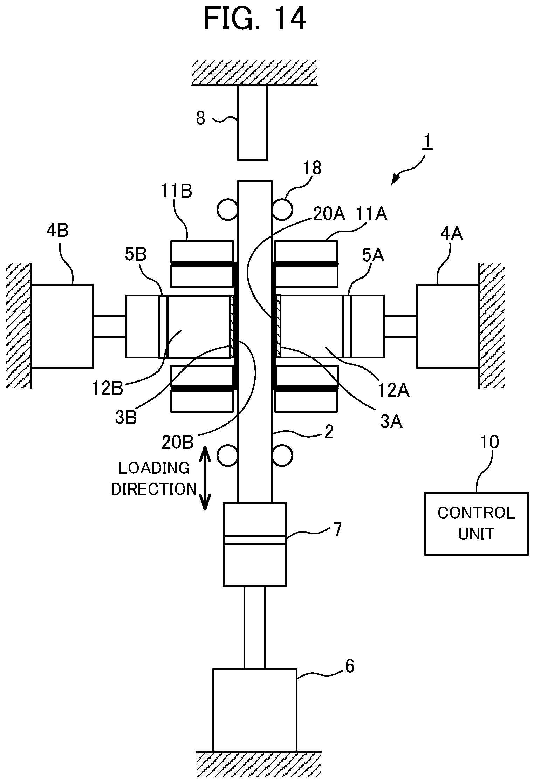

FIG. 14 is a view illustrating a configuration of a slip test device according to Embodiment 6 of the present disclosure. The slip test device 1 according to Embodiment 6 is configured by combining the configuration of Embodiment 5 with the pressing mechanism of Embodiment 4. As in Embodiment 5, the test member 2 is supported in such a manner that the test member 2 is movable in the horizontal direction.

As in Embodiment 4, the slip test device 1 according to Embodiment 6 includes the two slip sensors 3A and 3B that face each other with the test member 2 interposed between the slip sensors 3A and 3B. The slip test device 1 includes two holding parts 11A and 11B that respectively correspond to the slip sensors 3A and 3B and that respectively hold the samples 20A and 20B. In the configuration illustrated in FIG. 14, the pressing mechanism includes the two pressing machines 4 A and 4 B, the two pressing force meters 5A and 5B, and the two pressing parts 12A and 12B, the sample 20A and the slip sensor 3A are pressed against one end surface of the test member 2 in the direction perpendicular to the loading direction, and the sample 20B and the slip sensor 3B are pressed against the other end surface of the test member 2 in the direction perpendicular to the loading direction.

In the slip test device 1 according to Embodiment 6, although the holding parts 11A and 11B can freely move in the horizontal direction, the holding parts 11A and 11B are desirably supported so as not to fall under their own weight. Also in Embodiment 6, the holding parts 11A and 11B may be configured to apply tension to the samples 20A and 20B.

Also, the slip test device according to Embodiment 6 may use two racks that are parallel to and opposite to each other and a pinion with which the two racks mesh to move in directions opposite to each other. The slip test device 1 of Embodiment 6 may have a configuration in which the pressing parts 12A and 12B are fixed to the racks that move in directions opposite to each other, the racks are moved by a pinion, and the test member 2 is sandwiched between the pressing parts 12A and 12B. In a case in which the pressing mechanism includes the racks and the pinion, the pressing mechanism may be configured in such a manner that the whole of the pressing mechanism including the motor, the racks and the pinion can freely move in the direction of the pressing force.

Since the test member 2 does not move unless a load is applied to the test member 2, the process of the slip test in Embodiment 6 is the same as that in Embodiment 4. Also in the slip test device 1 of Embodiment 6, as in the process according to Embodiment 3 illustrated in FIG. 8, the step of measuring displacement and the sliding motion can be repeatedly performed while the pressing force is being decreased.

Also in Embodiment 6, the loading machine 6 may apply, to the test member 2, not only the force in the loading direction but also a torque for rotating the test member 2 around an axis in the loading direction. In that case, the test member 2 is desirably a cylinder. A portion of the test member 2 that comes into contact with the support portion 16 is at least formed to be a cylindrical surface. The support portion 16 comes into contact with the test member 2 does not come into contact with a roller 17 but comes into contact with a ball that can rotate in all directions, to maintain the position of the test member 2 in the vertical direction.

Also in Embodiment 6, as in Embodiment 2, the sample displacement meter 21 can be used instead of at least one of the slip sensors 3A and 3B. Alternatively, the slip sensor 3A or 3B and the sample displacement meter 21 may be used in combination. Alternatively, the sliding motion of the sample 20A or 20B relative to the pressing mechanism may be measured by the sample displacement meter 21, and the sliding motion of the sample 20A or 20B relative to the test member 2 may be measured by the slip sensor 3A or 3B with the slip sensor 3A or 3B fixed to the test member 2. In this case, the slip sensor 3A or 3B fixed to the test member 2 can be regarded as a displacement meter that measures the movement of the test member 2 in the loading direction. The sample displacement meter 21 is a slip measurer that measures the sliding motion of the sample 20A or 20B relative to the pressing mechanism.

The present disclosure is not limited to the above-described embodiments. The holding part 11 may be fixed to the pressing part 12. In this case, the slip sensor 3 measures elongation of the sample 20 in the loading direction as a sliding motion of the sample 20 relative to the pressing mechanism or the test member 2 in the loading direction.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

### Reference Signs List

- 1: Slip test device
- 2: Test member
- 3, 3A, 3B: Slip sensor
- 4, 4A, 4B: Pressing machine
- 5, 5A, 5B: Pressing force meter
- 6: Loading machine
- 7: Load meter
- 8: Displacement meter
- 9: Opposite unit
- 10: Control unit
- 11, 11A, 11B: Holding part
- 12, 12A, 12B: Pressing part
- 13: Wire
- 14: Weight
- 15: Pulley
- 16: Support portion
- 17, 18: Roller
- 20, 20A, 20B: Sample
- 21: Sample displacement meter
- 23: Wire
- 24: Weight
- 25: Pulley

## Claims

1. A slip test device comprising:
a test member;
a pressing mechanism whose movement in a loading direction of the test member is constrained, the pressing mechanism being configured to press a sample against an end surface of the test member that is an end portion of the test member in a direction perpendicular to the loading direction with the sample sandwiched between the end surface of the test member and the pressing mechanism;
a holding part to hold the sample between the end surface of the test member and the pressing mechanism;
a pressing force meter to measure a pressing force with which the pressing mechanism presses the sample against the end surface of the test member;
a displacement meter to measure the movement of the test member in the loading direction; and
a slip measurer to measure a sliding motion of the sample in the loading direction relative to the pressing mechanism or the test member.

2. The slip test device according to claim 1, wherein the holding part holds the sample between the end surface of the test member and the pressing mechanism in such a manner that the sample is movable relative to the test member and the pressing mechanism in the loading direction.

3. The slip test device according to claim 1 or 2, the slip test device comprising a control unit configured to:
cause the pressing mechanism to apply, to a boundary portion between the end surface and the sample, the pressing force having such magnitude as does not cause the test member to move in the loading direction;
cause the displacement meter to measure the movement of the test member in the loading direction and cause the slip measurer to measure the sliding motion of the sample relative to the test member and the pressing mechanism, while decreasing the pressing force applied by the pressing mechanism.

4. The slip test device according to claim 3, wherein
when a predetermined condition is met after a start of the measurement of the movement of the test member in the loading direction and a start of the measurement of the sliding motion of the sample relative to the pressing mechanism or the test member, the control unit
(i) causes the pressing mechanism to again apply, to the boundary portion between the end surface and the sample, the pressing force having such magnitude as does not cause the test member to move in the loading direction, and
(ii) causes the displacement meter to again measure the movement of the test member in the loading direction and causes the slip measurer to again measure the sliding motion of the sample relative to the pressing mechanism or the test member, while decreasing the pressing force again applied by the pressing mechanism.

5. The slip test device according to any one of claims 1 to 4, the slip test device comprising:
a supporting mechanism supporting the test member in such a manner that the test member is movable in the loading direction perpendicular to a direction in which the pressing mechanism presses the sample; and
a loading machine to apply, to the test member, a force in the loading direction perpendicular to the direction in which the pressing mechanism presses the sample.

6. The slip test device according to claim 5, wherein the loading machine applies, to the test member, not only the force in the loading direction but also a torque for rotating the test member around an axis in the loading direction.

7. The slip test device according to claim 5 or 6, wherein
the supporting mechanism suspends and supports the test member with the loading direction kept vertical,
the pressing mechanism presses the sample and the slip measurer against the end surface of the test member in a horizontal direction, and
the displacement meter measures the movement of the test member in the vertical direction.

8. The slip test device according to any one of claims 1 to 7, wherein
the test member has a columnar shape and the loading direction is a direction of a central axis of the columnar shape,
the pressing mechanism presses the sample against a lateral surface of the test member in a direction perpendicular to the direction of the central axis of the columnar shape, and
the displacement meter measures the movement of the test member in the direction of the central axis of the test member.

9. The slip test device according to any one of claims 1 to 8, wherein the holding part comprises a mechanism for applying a tension to the sample in the direction perpendicular to the direction in which the pressing mechanism presses the sample.

10. The slip test device according to any one of claims 1 to 9, wherein the pressing mechanism comprises an opposite unit (i) facing the sample with the test member interposed between the opposite unit and the sample, (ii) allowing the test member to move in the loading direction, and (iii) maintaining a position of the test member in the direction perpendicular to the loading direction against the force with which the sample is pressed.

11. The slip test device according to any one of claims 1 to 9, wherein
the sample is two samples of a first sample and a second sample,
the pressing mechanism presses the first sample against one end surface of the test member in the direction perpendicular to the loading direction and presses the second sample against another end surface of the test member in the direction perpendicular to the loading direction,
the holding part comprises a first holding element to hold the first sample and a second holding element to hold the second sample, and
the slip measurer comprises a first slip measuring element to measure a sliding motion of the first sample in the loading direction relative to the pressing mechanism or the test member and a second slip measuring element to measure a sliding motion of the second sample in the loading direction relative to the pressing mechanism or the test member.

12. A slip test method comprising:
pressing a sample against an end surface of a test member in a direction perpendicular to a loading direction of the test sample using a pressing mechanism whose movement in the loading direction of the test member is constrained, the end surface being an end portion of the test member in the direction perpendicular to the loading direction;
measuring, using a pressing force meter, a pressing force with which the pressing mechanism presses the sample against the end surface of the test member; and
while decreasing the pressing force having such magnitude as does not cause the test member to move in the loading direction after the pressing force is applied to a boundary portion between the end surface and the sample by the pressing mechanism, measuring, using a displacement meter, the movement of the test member in the loading direction and measuring, using a slip measurer, a sliding motion of the sample in the loading direction relative to the pressing mechanism or the test member.
